# EUROPÄISCHE PATENTSCHRIFT

(11) **EP 1 433 432 B1**
(45) Veröffentlichungstag und Bekanntmachung des Hinweises auf die Patenterteilung: **11.05.2005**
(21) Anmeldenummer: 02028015.2
(22) Anmeldetag: 13.12.2002
(51) Int. Cl.: A61B 19/02, G06F 19/00

(54) **System zum Integrieren verschiedener medizinisch verwendbarer Geräte**
System for integrating various devices for medical applications
Système pour intégrer des dispositifs diverses pour applications médicaux

(43) Veröffentlichungstag der Anmeldung: 30.06.2004
(73) Patentinhaber: BrainLAB AG, 85551 Kirchheim/Heimstetten (DE)
(72) Erfinder: Birkenbach, Rainer, 85586 Poing (DE); Vilsmeier, Stefan, 6330 Kufstein (AT)
(74) Vertreter: Schwabe - Sandmair - Marx

(56) Entgegenhaltungen:
- WO-A-02/19957
- US-A- 5 788 688
- US-A- 6 117 127

## Beschreibung

Die vorliegende Erfindung bezieht sich auf ein System zum Integrieren verschiedener medizinisch verwendbarer elektrischer oder elektronischer Geräte. Insbesondere bezieht sich die Erfindung auf das einfache und kostengünstige Integrieren oder Zusammenschalten verschiedener Geräte, welche unterschiedliche Vorrichtungen, wie zum Beispiel Kameras, Sonden oder Pumpen ansteuern und zum Beispiel unterschiedliche Spezifikationen an den Schnittstellen zur Ein- und Ausgabe von Daten oder Steuersignalen haben.

Bei medizinischen Behandlungen, insbesondere bei chirurgischen Eingriffen, wird eine Vielzahl unterschiedlicher medizinischer Geräte mit verschiedenen Funktionen häufig gleichzeitig verwendet. Insbesondere bei offenen oder minimal invasiven chirurgischen Eingriffen werden zum Beispiel Bilder durch Laparoskope und Endoskope aufgenommen, welche von verschiedenen Herstellern stammen können und ein jeweils aufgenommenes Bild auf einem von dem Hersteller mitgelieferten Monitor nach entsprechender Bearbeitung der Aufnahmesignale in zugehörigen von dem Hersteller bezogenen Steuergeräten oder Recheneinheiten darstellen. Weiterhin werden bei chirurgischen Eingriffen zum Beispiel Geräte zum Überwachen des Kreislaufs oder des Stoffwechsels eines Patienten eingesetzt, um den Zustand des Patienten vor, während und nach einem chirurgischen Eingriff zu überwachen und gegebenenfalls Warnsignale zu erzeugen, falls beispielsweise die Atmung oder der Herzschlag Unregelmäßigkeiten zeigen oder Probleme bei der Anästhesie auftreten. Des weiteren werden je nach Art eines chirurgischen Eingriffs auch Fluoroskopie-, Röntgen-, Ultraschallgeräte, Kernspintomographen usw. verwendet, mit welchen Bilder erzeugt werden, die auf speziellen von den einzelnen Herstellern gelieferten Bildschirmen angezeigt werden. Weiterhin können je nach vorhandener Ausrüstung von einem Chirurgen unterschiedliche Einstellungen zum Beispiel der Patientenliege, des Lichtes, der Lautstärke von akustischen Ausgaben oder der Raumtemperatur vorgenommen werden. Im Allgemeinen weisen Arbeitsplätze für chirurgische Eingriffe, bei welchen verschiedene Geräte verwendet werden, eine Vielzahl von zu den jeweiligen Geräten gehörenden Steuervorrichtungen und eine Vielzahl von Anzeigeinstrumente auf, welche häufig möglichst gleichzeitig von einem Chirurgen während eines Eingriffs im Auge behalten werden sollen.

Aus der US 6,117,127 ist ein medizinisch-technischer Systemarbeitsplatz für die offene oder minimal invasive Chirurgie bekannt, wobei eine Ablageschale und mindestens eine Anschlusseinheit für Instrumente medizinisch-technischer Geräte, ein räumlich von der Anschlusseinheit getrenntes Gerätecenter zur Aufnahme der medizinisch-technischen Geräte und eine Verbindungseinheit vorgesehen ist, welche die Anschlusseinheit und das Gerätecenter miteinander verbindet.

Die US 6,471,363 B2 beschreibt eine Vorrichtung für chirurgische Zwecke, welche an der Decke eines Operationsraumes angebracht ist und verschiedene Kameras und Monitore aufweist, welche relativ zueinander verschwenkt werden können.

Die US 5,788,688 beschreibt ein Steuersystem für Chirurgen mit einer unabhängigen PC-basierten elektronischen Steuereinheit, welche es ermöglich, dass verschiedene Instrumente, welche bei der chirurgischen Endoskopie verwendet werden, einheitlich verwendet werden können. Gemäß einer Ausführungsform werden verschiedene elektrisch betriebene Geräte so zusammengeschaltet, dass die Bedienung dieser unterschiedlich betriebenen Geräte vereinheitlicht wird.

Allgemein besteht bei den bekannten sogenannten integrierten Systemen zum gleichzeitigen Betreiben einer Vielzahl unterschiedlicher Geräte das Problem, dass entweder alle Systeme von einem einzigen Hersteller bezogen werden müssen und somit eventuell bereits vorhandene Geräte oder Systeme nicht einfach integriert werden können, oder dass zum Integrieren von neuen Geräten in ein System unterschiedliche Ein- und Ausgabe-Spezifikationen das Integrieren erschweren, da herstellerspezifische Protokolle verwendet werden, so dass die Software geändert werden muss, wenn ein Gerät mit einem anderen zusammengeschaltet werden soll und da häufig verschiedene Bedienkonzepte von verschiedenen Herstellern gewählt werden.

Es ist eine Aufgabe der vorliegenden Erfindung ein System zum Integrieren oder Verbinden von mindestens zwei oder allgemein einer Mehrzahl von medizinischen Geräten vorzuschlagen, welches das kostengünstige und einfache Integrieren oder Zusammenschalten von Geräten mit unterschiedlichen Spezifikationen ermöglicht.

Diese Aufgabe wird durch das System gemäß Anspruch 1 gelöst. Vorteilhafte Ausführungsformen ergeben sich aus den abhängigen Ansprüchen.

Das erfindungsgemäße System zum Verbinden von mindestens zwei oder allgemein einer Mehrzahl von medizinischen elektrischen oder elektronischen Geräten weist eine zentrale Steuereinheit auf, mit welcher die Eingabe- und Ausgabeanschlüsse verschiedener Steuergeräte unterschiedlicher medizinischer Instrumente oder Geräte verbunden werden können. Dabei bleiben erfindungsgemäß die medizinischen Geräte, wie zum Beispiel Endoskope, Laparoskope, Pumpen, Ultraschallköpfe, Bohrer, Kameras oder Kamerasysteme und dergleichen mit ihren jeweiligen Steuergeräten verbunden, so dass es nicht erforderlich ist in den internen Ablauf der Ansteuerung eines medizinischen Gerätes einzugreifen. Mit anderen Worten muss die Software eines Steuergerätes zum Betreiben eines medizinischen Instruments oder Geräts nicht modifiziert werden, so dass kein großer Aufwand zum erfindungsgemäßen Integrieren eines Steuergeräts für ein medizinisches Instrument oder Gerät in ein System, weder bezüglich der Hardware, noch bezüglich der Software erforderlich ist. Erfindungsgemäß werden die an den Steuergeräten der einzelnen medizinischen Instrumente oder Geräte vorgesehenen Eingabe- und Ausgabeanschlüsse zum Beispiel zum Anschließen einer Tastatur, einer Cursor-Steuervorrichtung wie zum Beispiel einer Maus, oder eines Bildschirms mit der zentralen Steuereinheit verbunden, wodurch die zentrale Steuereinheit lediglich die als Eingabe- und Ausgabe-Schnittstelle zu einem Benutzer des medizinischen Instruments oder Geräts erforderlichen Daten bzw. Signale erfasst und weiterverarbeitet, um Daten von einem oder mehreren medizinischen Instrumenten oder Geräten beispielsweise auf einem einzigen Bildschirm darzustellen. Ebenso kann eine zentrale Eingabeeinheit, wie beispielsweise eine Tastatur, ein Joystick, eine Maus, ein als Touch-Screen ausgebildeter Bildschirm, ein Mikrofon zum Erfassen von Sprachsignalen, ein mit der Hand oder mit dem Fuß betätigbarer Schalter oder ähnliches mit der zentralen Steuereinheit verbunden sein, um so von einem Benutzer eingegebene Signale oder Steuerinformationen über die zentrale Steuereinheit zu erfassen und zum Beispiel in Abhängigkeit von einer vorherigen Auswahl durch den Benutzer an ausgewählte Steuergeräte der medizinischen Instrumente oder Geräte weiterzugeben, wodurch die Einstellung oder die Arbeitsweise eines oder mehrerer medizinischer Geräte oder Instrumente verändert werden kann.

Vorteilhaft kann auch eine Videokamera oder ein Kamerasystem vorgesehen sein, um Bilddaten zum Beispiel für Videokonferenzen zu übertragen oder zu Dokumentationszwecken zu speichern. Eine solche Kamera kann zum Beispiel auch als Webcam verwendet werden.

Indem nur die zur Interaktion mit einem Benutzer erforderlichen Signale der Steuergeräte der medizinischen Instrumente an die zentrale Steuereinheit weitergeleitet bzw. von dieser zentralen Steuereinheit an die einzelnen Steuergeräte weitergeleitet werden, kann jedes einzelne Gerät ohne Modifikation der Software oder der Hardware so verwendet werden, wie es von dem jeweiligen Hersteller konzipiert wurde, wodurch das mit dem einzelnen Steuergerät verbundene medizinische Instrument weiterhin so angesteuert wird, wie dies in dem zugehörigen Steuergerät von dem jeweiligen Hersteller spezifiziert ist. Erfindungsgemäß wird die für den Benutzer wichtige Anzeige von Bildern, Signalen oder Betriebszuständen der medizinischen Instrumente, insbesondere werden die Ausgabesignale der Steuergeräte, durch die zentrale Steuereinheit erfasst und an einer zentralen Anzeige, wie zum Beispiel einem Flachbildschirm dargestellt, wobei durch eine zentrale Eingabevorrichtung ein oder mehrere Steuergeräte und damit medizinische Instrumente ausgewählt werden können und weiterhin diesen Steuergeräten der jeweiligen medizinischen Instrumente Signale zur Bestimmung oder Änderung der Betriebsart oder Arbeitsweise von der zentralen Eingabevorrichtung über die zentrale Steuereinheit übertragen werden können.

Somit kann erfindungsgemäß im Operationsbereich die häufig große Anzahl von Monitoren und Steuergeräten mit häufig unterschiedlichen Bedienkonzepten verringert werden und es kann beispielsweise ein einziger Monitor mit einer zentralen Eingabevorrichtung zur Realisierung eines einheitliche Bedienkonzeptes verwirklicht werden, wobei verschiedene Steuergeräte unterschiedlicher medizinischer Instrumente angesteuert werden können, ohne zum Beispiel die in den Steuergeräten verwendete Software umzuschreiben oder ohne dass dem Hersteller eines Steuergeräts ein spezielles Protokoll für die Kommunikation mit der zentralen Steuereinheit vorgegeben werden muss.

In der erfindungsgemäß vorgesehenen zentralen Steuereinheit kann zum Beispiel eine Einheit zur Verarbeitung von Videosignalen unterschiedlicher Formate vorgesehen sein, mit welcher zum Beispiel Videosignale unterschiedlicher Formate für die für die jeweiligen Steuergeräte ursprünglich vorgesehenen Monitore verarbeitet und in ein bestimmtes ausgewähltes Videosignal-Format umgesetzt werden können, so dass die von den unterschiedlichen Steuergeräten ausgegebenen Videosignale mit möglicherweise unterschiedlichen Formaten an zum Beispiel einem einzigen mit der zentralen Steuereinheit verbundenen Monitor dargestellt werden können. Verfahren und Vorrichtungen zum Umsetzen von Videosignalen eines bestimmten Formats in ein anderes Format, wie zum Beispiel PAL in SECAM oder andere Formate sind im Stand der Technik bekannt und werden hier nicht näher beschrieben.

Die zur Eingabe von Steuersignalen vorgesehenen Anschlüsse an Steuergeräten sind häufig zum Anschließen einer kommerziell erhältlichen Tastatur, Maus, eines Joysticks, eines Trackballs oder ähnlichem standardisiert und können somit im Falle von standardisierten Anschlüssen zum Beispiel einfach mit der erfindungsgemäß vorgesehenen zentralen Steuereinheit verbunden werden. In der erfindungsgemäß vorgesehenen zentralen Steuereinheit kann auch ein Prozessor vorgesehen sein, welcher die Umsetzung von Eingabesignalen eines bestimmten Formats in ein anderes Format erlaubt. Beispielsweise kann der Prozessor so ausgebildet sein, dass die über einen Touchscreen auf einer dort angezeigten Tastatur eingegebenen Signale umgesetzt werden in Steuersignale, welche dem Tastaturanschluss eines Steuergeräts eingegeben werden. Ebenso können die Signale verschiedener Cursor-Steuervorrichtungen, wie zum Beispiel Maus, Touchpad, Joystick, Trackball oder ähnliches in gewünschte andere Formate umgesetzt werden, so dass zum Beispiel ein mit der zentralen Steuereinheit verbundenes Eingabegerät zum Beispiel zur Bewegung eines Cursors Steuersignale erzeugen kann, welche von einem in der zentralen Steuereinheit vorgesehenen Prozessor in Steuersignale umgesetzt werden können, die von einem bestimmten Steuergerät eines medizinischen Instruments interpretiert werden können, so dass ein einheitliches Bedienkonzept zur Bedienung unterschiedlicher Steuergeräte verschiedener medizinischer Instrumente realisiert werden kann, obwohl die Steuergeräte ursprünglich zum Beispiel die Bedienung durch eine Tastatur für ein erstes Steuergerät, die Bedienung durch eine Maus für ein zweites Steuergerät und die Bedienung durch einen Touchscreen für ein drittes Steuergerät erforderlich machten.

Vorteilhaft ist das erfindungsgemäße System in Form eines Schrankes oder Racks ausgebildet, wobei Einschubfächer für zwei oder mehrere Steuergeräte vorgesehen sind, so dass ein, zwei oder mehrere in das Rack eingesetzte Steuergeräte lediglich mit den durch diese Steuergeräte angesteuerten oder betriebenen medizinischen Instrumenten verbunden werden müssen. Eine weitere Verbindung des Steuergerätes mit einem Operationsraum ist nicht erforderlich, da die Eingaben und Ausgaben des Steuergerätes über die erfindungsgemäß vorgesehene zentrale Steuereinheit geleitet werden, wodurch es nicht mehr erforderlich ist, dass mehrere Instrumente zur Ausgabe oder Eingabe von Signalen an einem chirurgischen Arbeitsplatz vorgesehen werden müssen, welche einerseits den Chirurgen bei seiner Arbeit behindern können und möglicherweise aufgrund unterschiedlicher Bedienkonzepte verwirrend sind und welche andererseits die Gesamtkosten eines chirurgischen Arbeitsplatzes erhöhen, da an einem solchen Arbeitsplatz vorgesehene Geräte strenge medizinische Standards erfüllen müssen. Durch die erfindungsgemäße Zusammenfassung der Eingabe- und Ausgabesignale und der Anzeige entsprechender Ausgabesignale an einer zentralen Anzeigeeinheit können die Gesamtkosten eines chirurgischen Arbeitsplatzes erheblich verringert werden. Allgemein kann die Erfindung auch angewendet werden, wenn die einzelnen medizinischen Instrumente nicht räumlich miteinander zum Beispiel durch ein Rack verbunden sind, solange das erfindungsgemäße Konzept der zentralen Erfassung von Ein- und Ausgabesignalen der einzelnen Steuergeräte angewandt wird.

Bevorzugt können die Komponenten Bildschirm, Touchscreen, Tastatur, Maus oder andere Eingabe- und Ausgabegeräte direkt mit der zentralen Steuereinheit verbunden sein, wobei es möglich ist, dass die jeweiligen Komponenten jeweils separat mit der zentralen Steuereinheit verbunden sind. Um die Anzahl der zur Verbindung dienenden Kabel zu verringern, können auch die entsprechenden Signale digitalisiert werden, sofern diese noch in nicht digitaler Form vorliegen und können dann als ein digitales Signal zum Beispiel über eine einzelne als Bus verwendete Leitung übertragen werden, wobei die digital übertragenen Signale am Ende der Leitung wieder in die ursprünglichen Signale umgewandelt werden können. Weiterhin ist es auch möglich einen Teil oder die gesamten zu übertragenden Signale verbindungslos zu übertragen, wie zum Beispiel durch Funk oder Infrarot.

Die erfindungsgemäß mit der zentralen Steuereinheit verbundene mindestens eine Anzeigevorrichtung und die mindestens eine zentrale Eingabevorrichtung zur Eingabe von Steuersignalen in die zentrale Steuereinheit sind über einen Bus, vorzugsweise zur Übertragung von Daten mit einer hohen Datenrate, mit der zentralen Steuereinheit verbunden.

Der Bus kann beispielsweise als eine Glasfaserleitung ausgebildet sein, um aus Sicherheitsgründen eine galvanische Trennung zwischen der zentralen Steuereinheit und den mit dieser Steuereinheit verbundenen Steuergeräten einerseits und der in der Arbeitsumgebung des Chirurgen stehenden Geräten andererseits durchzuführen, was aus Sicherheitsgründen häufig gewünscht wird.

Das erfindungsgemäße System weist eine Datenausgabevorrichtung und/oder einer Dateneingabevorrichtung auf. Die Datenausgabevorrichtung kann beispielsweise ein Bildschirm, vorzugsweise ein Flachbildschirm sein, welcher vorteilhaft auch als Touchscreen und damit auch als zentrale Eingabevorrichtung ausgebildet sein kann. Es ist auch möglich mehr als nur eine Datenausgabevorrichtung vorzusehen, wobei beispielsweise ein an der Wand eines Operationsraumes angebrachter Großbildschirm vorgesehen sein kann, um zum Beispiel Daten oder Ansichten wiederzugeben, welche ansonsten zum Beispiel nur durch einen eine Operation durchführenden Chirurgen oder eine ein Gerät bedienende Person erfasst werden können. Ein erfindungsgemäß verwendbarer Bildschirm kann zum Beispiel ein LCD-Schirm sein oder auf einer Plasma-Rückprojektion oder einer Frontprojektion basieren. Allgemein kann erfindungsgemäß jedes Anzeigegerät verwendet werden, welches bevorzugt zur Anwendung im medizinischen Bereich geeignet ist. Hierzu kann aufgrund von medizinischen Standards zum Beispiel vorgeschrieben sein, das die Anzeigevorrichtung eine sterile Bedienung ermöglicht.

Vorteilhaft sollte die Eingabevorrichtung zur Eingabe von Steuersignalen auch den Bedürfnissen einer sterilen Arbeitsumgebung entsprechend ausgelegt sein, das heißt es sollte beispielsweise für einen Chirurgen, welcher Handschuhe trägt, einfach möglich sein bestimmte Optionen oder Menüs auszuwählen und entsprechende Bedien- oder Steuerbefehle einzugeben. Besonders bevorzugt ist hierzu ein zur Anzeige vorgesehener Bildschirm als Touchscreen ausgebildet, so dass auf diesem Bildschirm beispielsweise verschiedene Menüs ausgewählt und angezeigt werden können und ein Chirurg einfach durch Berührung eines entsprechenden angezeigten Feldes auf dem Bildschirm ein bestimmtes medizinisches Instrument bzw. das zugehörige Steuergerät auswählen und bestimmte Einstellungen daran vornehmen kann.

Bevorzugt ist eine Speichereinheit vorgesehen, in welcher ein ausgewählter Teil oder auch alle Signale, welche von den mit der zentralen Steuereinheit verbundenen medizinischen Instrumenten ausgegeben werden, gespeichert werden. Weiterhin können auch alle von dem Benutzer vorgenommenen Einstellungen oder Eingaben in der Speichereinheit gespeichert werden, um zum Beispiel für Lehrzwecke oder zur Dokumentation eines chirurgischen Eingriffs entsprechende Daten zur Verfügung zu haben.

Vorteilhaft ist mindestens eine der das erfindungsgemäße System bildenden Komponenten oder Vorrichtungen an der Decke zum Beispiel eines Operationsraumes montiert. Beispielsweise kann eine Vorrichtung zur optischen Datenerfassung, wie zum Beispiel eine Kamera, eine Vergrößerungsvorrichtung, Haltevorrichtungen für medizinische Instrumente oder Geräte, Bildschirme, wie zum Beispiel ein Touchscreen, Dateneingabevorrichtungen, wie zum Beispiel eine Tastatur, ein Joystick oder andere Komponenten unmittelbar an der Decke montiert oder an an der Decke montierten Trägem angebracht sein oder von diesen gehalten werden. Dabei ist es insbesondere vorteilhaft, dass an der Decke montierte Geräte oder Instrumente bewegt werden können, wozu vorteilhaft verschwenkbare und/oder verdrehbare Arme mit einem oder mehreren Gelenken vorgesehen sind. Eine Deckenmontage verringert die im Operationsraum benötigte Standfläche und führt somit zu einer Verbesserung der Bewegungsmöglichkeit von sich in dem Operationsraum bewegenden Personen oder zu bewegenden oder zu verschiebenden am Boden stehenden Geräten oder Instrumenten.

Das erfindungsgemäße System ermöglicht es ein Verfahren zum gleichzeitigen, parallelen oder sequentiellen Betreiben von mindestens zwei medizinischen Instrumenten oder Geräten durch zuführen, wobei die Ausgabesignale der medizinischen Instrumente oder der mit den medizinischen Instrumenten verbundenen Steuergeräte zu einer zentralen Steuereinheit übertragen werden und wobei die Steuereinheit Eingabesignale an die medizinischen Instrumente oder das mit dem jeweiligen medizinischen Instrument verbundene Steuergerät überträgt. Vorteilhaft werden die Ausgabesignale der medizinischen Instrumente oder der damit verbundenen Steuergeräte von der zentralen Steuereinheit an eine zentrale Anzeigevorrichtung übertragen. Bevorzugt werden von einer zentralen Eingabeeinheit Daten oder Signale an die zentrale Steuereinheit übertragen, welche diese an ein oder mehrere Steuergeräte oder unmittelbar an medizinische Instrumente weiterleitet.

Vorteilhaft werden die Anzeigen oder Ausgabesignale der medizinischen Instrumente bzw. der mit diesen verbundenen Steuergeräten auf einer einzigen Anzeigevorrichtung, wie zum Beispiel einem Flachbildschirm dargestellt, wobei es möglich ist, die Anzeigen der verschiedenen medizinischen Instrumente in den einzelnen Instrumenten zugeordneten Fenstern darzustellen. Beispielsweise können die von einem Laparoskop aufgenommenen Bilder in einem ersten Fenster, die von einem Endoskop aufgenommenen Bilder in einem zweiten Fenster und die mit verschiedenen Instrumenten erfassten Daten bezüglich des Zustands des Patienten in einem dritten Fenster angezeigt werden.

Bevorzugt können die Ausgaben oder Anzeigen verschiedener Instrumente oder Geräte kombiniert werden, so dass beispielsweise die durch ein bildgebendes Verfahren aufgenommene Struktur eines Körpers oder Körperteil beispielsweise in einem Fenster dargestellt wird, wobei in diese Darstellung Daten von anderen Instrumenten zum Beispiel zur Navigation eingeblendet werden.

Vorteilhaft ist die Anzeige der Ausgabeinformationen der verschiedenen medizinischen Instrumente so ausgebildet, dass eine Menüfunktion realisiert werden kann, das heißt es kann über ein Menü beispielsweise zwischen den verschiedenen mit der zentralen Steuereinheit verbundenen Instrumenten ausgewählt werden und in einem dann erscheinenden für das jeweilige Instrument spezifischen Untermenü können weitere Einstellungen oder Änderungen einer Arbeitsweise des Instruments oder Geräts vorgenommen werden.

Bevorzugt wird im Fall des Eintritts von bestimmten vorher definierten Zuständen, wie zum Beispiel einem kritischen Zustand des Patienten oder Problemen bei der Anästhesie, ein akustisches und/oder optisches Warnsignal erzeugt, so dass beispielsweise die Anzeige eines den Gesundheitszustandes des Patienten überwachenden Instruments automatisch in den Vordergrund gebracht wird, falls sich der Gesundheitszustand aufgrund beispielsweise einer signifikanten Veränderung der Atmung oder des Herzschlages verändert, wodurch sichergestellt werden kann, dass für einen Patienten kritische Zustände aufgrund der Vielzahl von angezeigten Informationen nicht versehentlich übersehen werden.

Vorteilhaft können auch die Umgebungsbedingungen des chirurgischen Arbeitsplatzes verändert werden, wobei zum Beispiel die Helligkeit oder allgemein die Beleuchtung, die Lautstärke von akustischen Ausgaben, die Temperatur oder andere Parameter eingestellt werden können.

Die Erfindung wird nachfolgend anhand eines Ausführungsbeispiels unter Bezugnahme auf die beiliegenden Figuren beschrieben werden. Es zeigen:
- Fig. 1: eine schematische Darstellung des erfindungsgemäßen Systems; und
- Fig. 2: eine schematische Ansicht einer mit dem erfindungsgemäßen System realisierbaren Bildschirmausgabe.

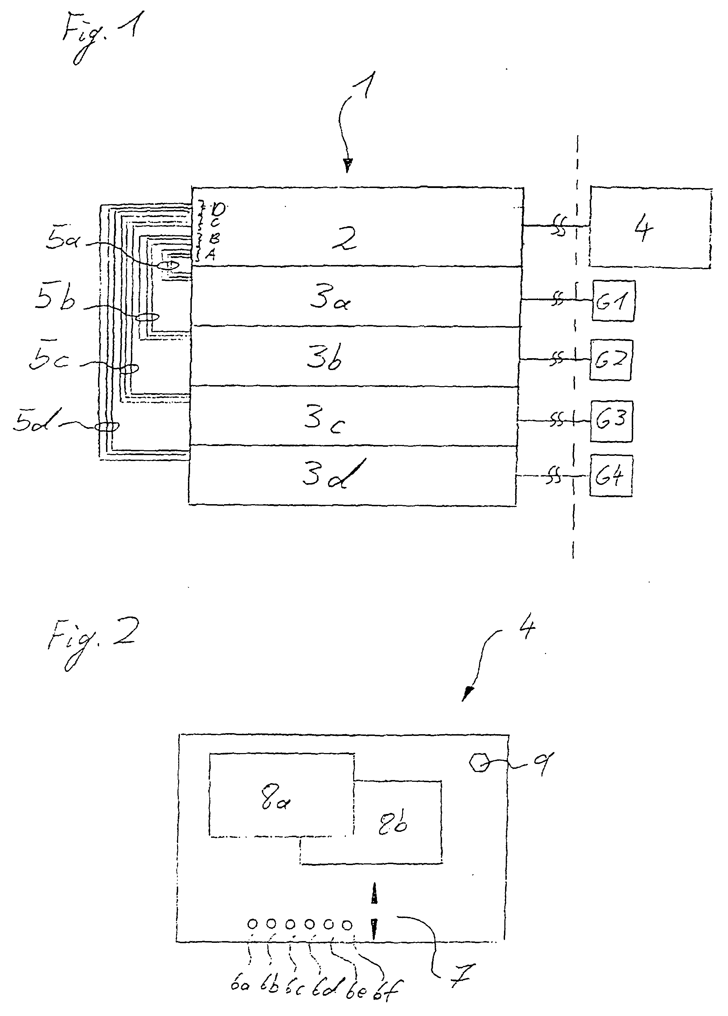

Figur 1 zeigt eine erfindungsgemäße Vorrichtung 1 mit einer zentralen Steuereinheit 2. Die zentrale Steuereinheit 2 weist die mit A, B, C und D bezeichneten Anschlüsse auf, an welchen die Leitungen 5a, 5b, 5c, und 5d angeschlossen sind, welche die zentrale Steuereinheit 2 mit den Steuergeräten 3a, 3b, 3c und 3d verbindet. Die Steuergeräte 3a, 3b, 3c und 3d sind mit medizinischen Instrumenten oder Geräten G1, G2, G3 und G4 verbunden. Beispiele solcher Instrumente können Endoskope, Laparoskope, ein Bohrgerät, Ultraschallgeräte, Infrarotgeräte, Kemspintomographen, Computertomographen, Pumpen für medizinische Substanzen oder ähnliches sein. Die Steuergeräte 3a bis 3d können von verschiedenen Herstellern stammen und unterschiedliche Bedienkonzepte aufweisen, um die damit verbundenen medizinischen Instrumente G1 bis G4 ansteuern und betreiben zu können. Die bei den jeweiligen Steuergeräten 3a bis 3d vorgesehenen Eingabe- und Ausgabeanschlüsse zum Beispiel für die Ausgabe von Messdaten oder Bildsignalen, oder für die Eingabe von Steuersignalen einer extern anschließbaren Vorrichtung zur Eingabe von Steuersignalen, wie zum Beispiel einer Tastatur oder einer Cursor-Steuervorrichtung, sind mit der zentralen Steuereinheit 2 verbunden, welche mindestens einen Prozessor aufweist, der Ausgabesignale der Steuergeräte 3a bis 3d in ein einheitliches Format umsetzen kann, um diese auf einem einzigen Bildschirm 4 in Abhängigkeit von einem eingestellten Modus parallel oder einzeln auszugeben. Der Bildschirm 4 ist im Ausführungsbeispiel als Touchscreen ausgebildet und kann somit auch zur Eingabe von Steuersignalen verwendet werden, welche an die zentrale Steuereinheit 2 übertragen werden. In dieser zentralen Steuereinheit 2 wiederum können ein oder mehrere Prozessoren vorgesehen sein, um die vom Bildschirm 4 stammenden Steuersignale in den jeweiligen Steuergeräten 3a bis 3d entsprechende Formate umzusetzen, die über die Leitungen 5a bis 5d an die entsprechenden Steuergeräte 3a bis 3d weitergeleitet werden können. Somit kann der Bildschirm 4 als zentrale Anzeige- und Steuervorrichtung zur Bedienung einer Vielzahl von unterschiedlichen medizinischen Instrumenten G1 bis G4 dienen.

Die in Figur 1 gezeigte gestrichelte Linie zeigt die Abgrenzung der erfindungsgemäßen Vorrichtung 1 von einem Operationssaal an, in welchem sich der Bildschirm 4 zusammen mit den medizinischen elektrischen oder elektronischen Instrumenten G1 bis G4 befindet. Die erfindungsgemäße Vorrichtung 1 kann außerhalb des Operationsbereiches aufgestellt werden und muss somit nicht die speziellen medizinischen Standards erfüllen, welche für im Operationsbereich verwendete Geräte gelten. Die Verwendung nur eines einzigen Bildschirms 4 zur Ansteuerung einer Vielzahl von medizinischen Instrumenten gestaltet den Operationsbereich übersichtlicher und ermöglicht somit auch eine größere Bewegungsfreiheit im Vergleich zu bisher bekannten Anordnungen mit einer Vielzahl von Anzeigevorrichtungen. Allgemein können statt der beispielhaft gezeigten einen Anzeigevorrichtung 4 auch zwei oder mehr Anzeigevorrichtungen vorgesehen werden, um zum Beispiel auch einen Assistenten eines Arztes oder Chirurgen Informationen anzeigen zu können.

Figur 2 zeigt eine beispielhafte Darstellung auf der in Figur 1 gezeigten Anzeigevorrichtung 4. Die Anzeigevorrichtung 4 ist als Tochscreen ausgebildet und zeigt im unteren Bereich Elemente 6a bis 6f an, über welche beispielsweise die medizinischen Instrumente G1 bis G4 oder einzelne Menüpunkte eines ausgewählten Menüs angewählt werden können. Die beispielhaft als nach oben und unten weisende Pfeilspitzen dargestellten Symbole können zum "Blättern" in einem Menü, zur Einstellung oder zur Veränderung von Werten verwendet werden, um zum Beispiel die Helligkeit einer Lampe zu erhöhen oder zu verringern. In den beispielhaft dargestellten Fenstern 8a und 8b des Bildschirmes 4 können die Videobilder von zwei verschiedenen Kameras dargestellt werden, wobei zum Beispiel für jedes einzelne medizinische Instrument ein oder mehr Fenster geöffnet werden können. Die Darstellung einer Vielzahl von unterschiedlichen Informationen oder Programmen durch Fenster, welche sich auf einem Bildschirm überlappen können, ist im Stand der Technik bekannt und wird demzufolge hier nicht näher beschrieben.

Eine Alarmierungsanzeige 9 ist an einem festen Platz der Anzeigevorrichtung 4 vorgesehen, wobei die Anzeige 4 vorteilhaft so angesteuert wird, dass die Alarmierungsanzeige 9 nicht durch Fenster 8a oder 8b verdeckt werden kann. Die Alarmierungsanzeige 9 kann zum Beispiel ein leicht zu erfassendes blinkendes Signal ausgeben, falls über eines der medizinischen Instrumente G1 bis G4 festgestellt wird, dass sich der Zustand eines Patienten verändert oder kritisch wird.

## Patentansprüche

1. System mit einer Vorrichtung zum Verbinden von mindestens zwei medizinisch verwendbaren Instrumenten (G1, G2, G3, G4) mit einer zentralen Steuereinheit (2), wobei die mindestens zwei medizinisch verwendbaren Geräte (G1-G4) mit mindestens einem Steuergerät (3a, 3b, 3c, 3d) verbunden sind und die Eingabeund/oder Ausgabeanschlüsse der Steuergeräte (3a-3d) mit der zentralen Steuereinheit (2) verbunden sind und mit einer zentralen Eingabevorrichtung und einer Datenausgabevorrichtung, wobei die zentrale Eingabevorrichtung und die Datenausgabevorrichtung über einen Bus mit der zentralen Steuereinheit (2) verbunden sind, **dadurch gekennzeichnet, dass** der Bus eine galvanische Trennung zwischen der zentralen Eingabevorrichtung und/oder der Datenausgabevorrichtung einerseits und der zentralen Steuereinheit (2) andererseits gewährleistet.

2. System nach Anspruch 1, wobei die zentrale Steuereinheit (2) mindestens einen Prozessor aufweist, mit welchem verschiedene Anzeigeinformationen und/oder Bildformate in ein vorgegebenes definiertes Bildformat umgesetzt werden können.

3. System nach einem der vorhergehenden Ansprüche, wobei die zentrale Steuereinheit (2) mindestens einen Prozessor aufweist, mit welchem Steuersignale, welche der zentralen Steuereinheit (2) eingegeben werden, in weitere Steuersignale zur Ansteuerung von Steuergeräten (3a-3d) zur Steuerung von medizinischen Geräten (G1-G4) umgesetzt werden können.

4. System nach einem der vorhergehenden Ansprüche, wobei die mindestens zwei Steuergeräte (3a-3d) zur Ansteuerung der mindestens zwei medizinischen Geräte (G1-G4) in einem Rack vorgesehen sind.

5. System nach einem der vorhergehenden Ansprüche, wobei die Eingabevorrichtung ein Touchscreen, eine Tastatur, eine Cursor-Steuereinheit, eine Maus, eine Joystick, ein Trackball, ein Schalter, insbesondere ein Fußschalter, ein Touchpad oder eine Spracheingabevorrichtung ist.

6. System nach einem der vorhergehenden Ansprüche mit einer Speichereinheit zum Speichern von den medizinischen Instrumenten (G1-G4) erfassten Daten und/oder von über die Dateneingabevorrichtung eingegebenen Daten.

7. System nach einem der vorhergehenden Ansprüche, wobei mindestens eine der das System bildenden Komponenten oder Vorrichtungen an einer Decke montiert ist.

## Claims

1. A system comprising a device for coupling at least two medically applicable instruments (G1, G2, G3, G4) to a central control unit (2), wherein said at least two medically applicable apparatus (G1-G4) are coupled to at least one control apparatus (3a, 3b, 3c, 3d) and the input and/or output connections of said control apparatus (3a-3d) are coupled to said central control unit (2) and to a central input device and a data output device, wherein said central input device and said data output device are coupled to the central control unit (2) via a bus, **characterised in that** said bus ensures an electrical separation between the central input device and/or the data output device on the one hand and the central control unit (2) on the other hand.

2. The system as set forth in claim 1, wherein the central control unit (2) comprises at least one processor, using which different display information and/or image formats can be converted into a predetermined, defined image format.

3. The system as set forth in any one of the preceding claims, wherein the central control unit (2) comprises at least one processor, using which control signals inputted to the central control unit (2) can be converted into other control signals for controlling control apparatus (3a-3d) for controlling medical apparatus (G1-G4).

4. The system as set forth in any one of the preceding claims, wherein the at least two control apparatus (3a-3d) for controlling the at least two medical apparatus (G1-G4) are provided in a rack.

5. The system as set forth in any one of the preceding claims, wherein said input device is a touch screen, a keyboard, a cursor control unit, a mouse, a joystick, a trackball, a switch - in particular a foot switch - a touch pad or a speech input device.

6. The system as set forth in any one of the preceding claims, comprising a storage unit for storing data captured by the medical instruments (G1-G4) and/or data inputted via the data input device.

7. The system as set forth in any one of the preceding claims, wherein at least one of the components or devices forming the system is mounted to a ceiling.

## Revendications

1. Système qui présente un dispositif destiné à relier au moins deux instruments utilisables en médecine (G1, G2, G3, G4), qui présente une unité centrale de commande (2), les au moins deux appareils utilisables en médecine (G1 - G4) étant reliés à au moins un appareil de commande (3a, 3b, 3c, 3d) et les raccords d'introduction et/ou les raccords de sortie des appareils de commande (3a - 3d) étant reliés à l'unité centrale de commande (2), et qui présente un dispositif central d'introduction et un dispositif de sortie de données, le dispositif central d'introduction et le dispositif de sortie de données étant reliés à l'unité centrale de commande (2) par l'intermédiaire d'un bus, **caractérisé en ce que** le bus assure une séparation galvanique entre le dispositif central d'introduction et/ou le dispositif de sortie de données, d'une part, et l'unité centrale de commande (2), d'autre part.

2. Système selon la revendication 1, dans lequel l'unité centrale de commande (2) présente au moins un processeur qui permet de convertir différentes informations d'affichage et/ou différents formats d'image à un format d'image prédéfini.

3. Système selon l'une des revendications précédentes, dans lequel l'unité centrale de commande (2) présente au moins un processeur qui permet de convertir des signaux de commande introduits dans l'unité centrale de commande (2) en d'autres signaux de commande pour commander des appareils de commande (3a - 3d) destinés à commander des appareils médicaux (G1 - G4).

4. Système selon l'une des revendications précédentes, dans lequel les au moins deux appareils de commande (3a - 3d) destinés à commander les au moins deux appareils médicaux (G1 - G4) sont prévus dans un rack.

5. Système selon l'une des revendications précédentes, dans lequel le dispositif d'introduction est un écran tactile, un clavier, une unité de commande à curseur, une souris, une manette, une boule roulante, un commutateur, en particulier un commutateur à pédale, une plaque tactile ou un dispositif de commande vocale.

6. Système selon l'une des revendications précédentes, qui présente une unité de mémoire destinée à conserver des données saisies par les instruments médicaux (G1 - G4) et/ou des données introduites par l'intermédiaire du dispositif d'introduction de données.

7. Système selon l'une des revendications précédentes, dans lequel au moins l'un des composants ou dispositifs qui forment le système est fixé sur un plafond.
